(19) Europäisches Patentamt / European Patent Office / Office européen des brevets

(11) **EP 3 893 746 B1**

(12) **EUROPEAN PATENT SPECIFICATION**

(45) Date of publication and mention
of the grant of the patent:
**24.05.2023  Bulletin 2023/21**

(21) Application number: **19813561.8**

(22) Date of filing: **09.12.2019**

(51) International Patent Classification (IPC):
**A61B 5/053** (2021.01)     **A61B 5/16** (2006.01)
**A61B 5/00** (2006.01)

(52) Cooperative Patent Classification (CPC):
**A61B 5/0533; A61B 5/165; A61B 5/168;
A61B 5/486;** A61B 5/375; A61B 5/7282

(86) International application number:
**PCT/EP2019/084133**

(87) International publication number:
**WO 2020/120355 (18.06.2020 Gazette 2020/25)**

(54) **DEVICE, SYSTEM AND METHOD FOR PROVIDING BIO-FEEDBACK TO A USER**

VORRICHTUNG, SYSTEM UND VERFAHREN ZUR BEREITSTELLUNG VON BIOFEEDBACK FÜR EINEN BENUTZER

DISPOSITIF, SYSTÈME ET PROCÉDÉ POUR FOURNIR UN FEEDBACK BIOLOGIQUE À UN UTILISATEUR

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **11.12.2018  EP 18211570**

(43) Date of publication of application:
**20.10.2021  Bulletin 2021/42**

(73) Proprietor: **Koninklijke Philips N.V.
5656 AG Eindhoven (NL)**

(72) Inventor: **OUWERKERK, Martin
5656 AE Eindhoven (NL)**

(74) Representative: **Philips Intellectual Property &
Standards
High Tech Campus 52
5656 AG Eindhoven (NL)**

(56) References cited:
EP-A1- 3 409 205        WO-A1-2017/178359
GB-A- 2 440 751         US-A1- 2014 288 401
US-A1- 2017 000 398

**Description**

FIELD OF THE INVENTION

The present invention relates to a device, system and method for providing bio-feedback to a user.

BACKGROUND OF THE INVENTION

The short term habituation to recently experienced stimuli disrupts brain homeostasis. Maintaining attentional capacity then requires more energy. Normally sleep promotes dishabituation and the restoration of brain homeostasis, but the plethora of information the society offered to its inhabitants these days causes the disruption of homeostasis to occur at a time of the day where sleep is not an option.

There is a need for way that enables a user to restore brain homeostasis in a simple and efficient manner and to provide bio-feedback to the user, particularly regarding the restoration.

US 2014/0288401 A1 discloses a mental balance or imbalance estimation system and a method for estimating a level of mental balance or imbalance of a user. The system comprises a skin conductance sensor for sensing the skin conductance of the user, the skin conductance over time forming a skin conductance trace. The system further comprises a processing unit for receiving and processing the skin conductance trace, the processing unit configured to determine at least one stimulus response in the skin conductance trace, to determine an estimated cortisol level trace of the user based on the determined at least one stimulus response and to determine the estimated level of mental balance or imbalance of the user based on the estimated cortisol level trace.

SUMMARY OF THE INVENTION

It is an object of the present invention to provide a device, system and method that enable a user to restore brain homeostasis and that provide bio-feedback to the user.

In a first aspect of the present invention, defined in claim 1, a device for providing bio-feedback to a user is presented comprising

an interface configured to obtain skin conductance measurements of a user and to output user instructions and bio-feedback information; and
a processing unit configured to

- generate user instructions indicating one or more actions to follow by the user,
- identify one or more galvanic skin response (GSR) storms in a skin conductance trace of the obtained skin conductance measurements, wherein a GSR storm is identified based on the number of peaks and/or the peak frequency in a time period having a duration in the range of 2 to 6 minutes,
- identify a latency of a predetermined duration without a further GSR storm after the end of an identified GSR storm, and
- generate, if at least one GSR storm followed by a latency has been identified, bio-feedback information providing bio-feedback related to the one or more actions followed by the user.

In a further aspect of the present invention a system for providing bio-feedback to a user is presented comprising

- a sensing unit configured to acquire skin conductance measurements of the user,
- a device as disclosed herein configured to generate user instructions and to generate bio-feedback information based on the skin conductance measurements, and
- a user interface configured to output the user instructions and the bio-feedback information.

In yet further aspects of the present invention, there are provided a corresponding method, defined in claim 12, and a computer program, defined in claim 13, which comprises program code means for causing a computer to perform the steps of the method disclosed herein when said computer program is carried out on a computer as well as a non-transitory computer-readable recording medium that stores therein a computer program product, which, when executed by a processor, causes the method disclosed herein to be performed.

Preferred embodiments of the invention are defined in the dependent claims. It shall be understood that the claimed method, system, computer program and medium have similar and/or identical preferred embodiments as the claimed system, in particular as defined in the dependent claims and as disclosed herein.

The present invention is based on the idea enable or at least facilitate dishabituation and restoration of attentional capacity based on meditation, preferably based on the Zen meditation technique. Using skin conductance slow wave patterns caused by sympathetic nervous system (SNS) activity are used to coach the person into a meditative state known, e.g. in yoga for millennia, as the clear mind state, and described already in the ancient Indian document called Aphorisms of Patanjali. Thus, the attentional capacity is restored without the need for slow wave sleep. Further, bio-feedback information is generated based on the skin conductance measurements and provided to the user, e.g. to inform the user of the achievement of the clear mind state or the level of the achieved clear mind state.

In an embodiment the processing unit is configured to generate a meditation depth information indicating if at least one GSR storm followed by a latency has been identified as bio-feedback information. This helps the user to understand if a clear mind state has been achieved.

The processing unit is configured to identify a GSR storm in the skin conductance trace by detecting peaks in the skin conductance trace, in particular based on number of peaks and/or the peak frequency in a time period. This provides for a simple but reliable detection of a GSR storm. Preferably, the rising edge of peaks is detected. Rising edges composed of two overlapping peaks having only one top can be detected.

A GSR storm in the skin conductance trace may for instance be identified by detecting if the peak frequency of the peaks in the skin conductance trace exceeds a predetermined frequency threshold in the time period. A frequency in the range of 0.05 Hz to 1 Hz, in particular in the range of 0.1 Hz to 0.3 Hz, may hereby be used as predetermined frequency threshold.

A GSR storm in the skin conductance trace may, alternatively or additionally, be identified by detecting if the number of peaks in the skin conductance trace exceeds a predetermined threshold number in the time period. A number in the range of 3 to 60, in particular in the range of 6 to 18, may hereby be used as predetermined threshold number for a time period of one minute.

In another embodiment a duration in the range of 2 to 10 minutes, not according to the invention, or in particular in the range of 3 to 6 minutes, may be used as time period. Further, in an embodiment a minimum number of subsequent peaks may set to be shown in the skin conductance trace to qualify as GSR storm.

In a practical exemplary embodiment a threshold number may be set at 3 peaks/minute and a minimum number of 45 subsequent peaks may be shown in the skin conductance trace to qualify as a GSR storm.

The processing unit is further be configured to use a duration in the range of 2 to 10 minutes, in particular in the range of 3 to 6 minutes, as latency. This period may be set a as kind of waiting time after the end of a GSR storm before the bio-feedback information is output, e.g. signalling to the user that a clear mind state has reached.

In an embodiment user instructions may be generated that indicate one or more meditation actions to follow by the user. For instance, instructions how to sit, stand, lie, move, etc. may be issued as user instruction.

In another embodiment a meditation depth score may be generated based on the detection of peaks in the skin conductance trace, the meditation depth score indicating a meditation state of the user. The meditation depth score may e.g. indicate how deep and/or long the state of meditation is and/or how well and/or whether a clear mind state has been achieved and/or a brain homeostasis has been restored.

The system may be implemented as a wearable device, e.g. in the form of a wrist worn device or a body-mounted sensor. In an embodiment the sensing unit is a wearable skin conductance sensor, that measures at the upper side of the wrist (or any other skin part).

In another embodiment the user interface may comprise one or more of a display screen and/or a loudspeaker and/or a communication unit configured to communicate the user instructions and/or the bio-feedback information to another device, e.g. the user's smartphone, laptop or tablet. For instance, the user interface may be configured to convey the meditation depth score to the user or to an application or device that utilizes meditation depth information. More subtle information may be communicated using e.g. a built in buzzer. For instance, a buzz may indicate the presence of a GSR storm and three buzzes may indicate the clear mind state. Zen Buddhists use a bell for such signals, three for the start, one for intermediate signals and two as the end signal.

BRIEF DESCRIPTION OF THE DRAWINGS

These and other aspects of the invention will be apparent from and elucidated with reference to the embodiment(s) described hereinafter. In the following drawings

Fig. 1 shows a first example of a skin conductance trace, relative peak heights and an accelerometer output;
Fig. 2 shows a schematic diagram of an embodiment of a system and device according to the present invention;
Fig. 3 shows an exemplary implementation of a system according to the present invention in the form of a wearable device;
Fig. 4 shows a flow chart of an embodiment of a method according to the present invention; and
Fig. 5 shows a second example of a skin conductance trace, relative peak heights and an accelerometer output.

DETAILED DESCRIPTION OF THE EMBODIMENTS

In the digital age information and communication overload are commonplace. The individual is exposed to an increasing amount of information and communication. The brain perceives this as stimuli. Analogous to the homeostasis of the body the brain has mechanisms to maintain homeostasis when exposed to external influences. Information and communication stimuli cause effects in the brain which are recently linked to GluA1-dependent synaptic plasticity, and ultimately lower a person's attentional capability.

With time sleep pressure increases, the metabolic load to process new stimuli and information increases and the attentional capability decreases. Sleep, and more specifically slow wave sleep restores these negative effects. Slow wave sleep, also known as deep sleep or stage IV sleep, is characterized by delta range brain frequencies during which parts of the brain stop all neural activity. Sleep slow oscillations potentiate synapses that were depressed due to persistent activities during the previous day.

The information and communication overload linked to social media and other digital sources, such as computer games and online movies and videos, causes the negative effects to occur earlier in the day compared to the pre-digital age. The recovery of action potential of synapses that are impacted by these stimuli by something other than slow wave sleep would be very beneficial. During a Zen meditation parts of the brain turn silent. As will be shown below, similar skin conductance patterns can indeed be measured during slow wave sleep and during Zen meditation.

Emotions cause the activation sympathetic outflow of the autonomous nervous system. This activates among others the eccrine sweat glands. An apparatus that measures the conductance of the skin can be used to monitor emotions of a human being. The emotions cause skin conductance responses, which may be small peaks with a width of a few seconds. There are also skin conductance responses which cannot be linked to emotions, called non-specific skin conductance responses, which make up the GSR storms. Skin conductance (SC) is often referred to as electrodermal activity (EDA). For instance the polygraph is an apparatus that monitors emotions linked to lying using among others this principle.

Slow wave sleep causes a phenomenon in the skin conductance trace known as GSR storms. The intensity and duration of the GSR is generally higher when the previous day was more emotionally intense. This is in line with the brain homeostasis hypothesis discussed above. Hence, slow-wave sleep can be linked to sympathetic nervous system activity visible in the skin conductance trace. When skin conductance is measured at the wrist this is even more pronounced compared to skin conductance measured with traditional methods.

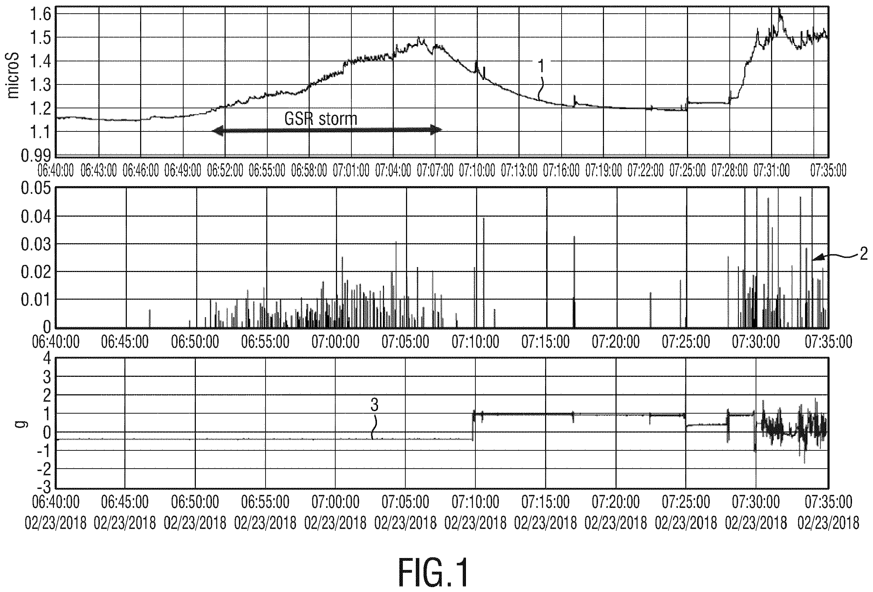

In Fig. 1 a skin conductance trace 1 (in $\mu$S over time) measured during sleep and showing a GSR storm is depicted. Further, Fig. 1 shows the relative peak heights 2 of the peaks in the skin conductance trace 1 and an accelerometer output signal 3. From these signals it can be derived that the sleep ends at 7:30 hrs. Further, the GSR storm appeared between 6:51 hrs and 7:07 hrs. The GSR storm is characterized by the occurrence of many small peaks with a frequency of about 0.5-1 Hz.

The delta brain wave frequency during slow wave sleep is just slow enough to may become visible as separate peaks in the fluctuations of the skin conductivity caused by the activity of the sympathetic nervous system. Whether there is a direct link between delta brain wave activity and the GSR storms is not proven yet. There is a coincidence of the two phenomena. Other sleep stages are linked to faster brain wave frequencies and thus cannot become visible in the skin conductivity fluctuations, since they fluctuate too fast. Apparently the skin conductance acts as a low pass filter, blocking all frequencies over e.g. 0.5 or 1 Hz, making it an excellent detector of slow (delta range) brain waves.

Fatigue caused by continuous information and communication processing can be relieved without the need for slow wave sleep. The diminished attentional capacity of a person can be restored without the need for slow wave sleep.

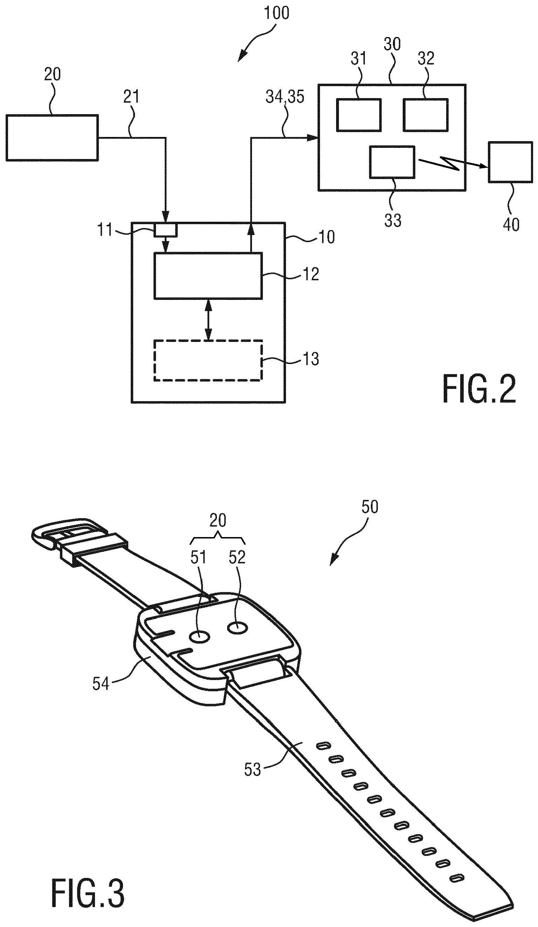

Fig. 2 shows a schematic diagram of exemplary embodiment of a system 100 according to the present invention. The system 100 comprises a sensing unit 20 for acquiring skin conductance measurements 21 of the user. The sensing unit 20 may e.g. a wearable skin conductance sensor. The skin conductance measurements 21 over time form a skin conductance trace. The system 100 further comprises a device 10 for generating user instructions 34 and for generating bio-feedback information 35 based on the skin conductance measurements 21. Still further, the system 100 comprises a user interface 30 for outputting the user instructions and the bio-feedback information. The user interface 30 may thus comprise one or more of a display screen 31, a signalling lamp, a loudspeaker 32 and/or a communication unit 33, which is configured to communicate the user instructions 34 and/or the bio-feedback information 35 to another device 40, such as a smartphone, laptop, table, computer, etc. of the user, where the user instructions and/or the bio-feedback information may be outputted and/or further processed.

The device 10 comprises an interface 11 for obtaining (i.e. receiving or retrieving from the sensor 20 or a memory (not shown)) the skin conductance measurements 21 of the user and for outputting the user instructions 34 and bio-feedback information 35 to the user interface 30. The device further comprises a processing unit 12 for processing the skin conductance measurements 21. The processing of the device and the corresponding method will be explained below in more detail.

The processing unit 12 can be any type of suitable processing unit or processor, such as for example a microprocessor/microcontroller, or embedded microcontroller but not limited thereto that is adapted accordingly. The interface 11 can be any kind of interface from obtaining data from the sensing unit 20 or a memory, e.g. a wireless or wired data interface or signal line. It will be understood that the sensing unit 20 and the device 10 can be part of the same device (e.g. wearable device or wristband) or can be implemented as or in separate devices.

It will be understood that the user interface 30 and the device 10 can be part of the same device (e.g. wearable device or wristband) or can be implemented as or in separate devices. For example, the user interface 30 of the system 100 may be implemented by means of a smartphone or other information processing entity at the same or a remote location. Correspondingly, the processing unit 12 can also be implemented by means of a smartphone that is adapted to perform the aforementioned functionality for example by running a corresponding application or another suitable computing device running the corresponding software.

The system 100 may further comprise a memory 13 for storing skin conductance measurements, user instructions and/or bio-feedback used earlier. The memory 13 can be part of the device 10 or can be an external memory. The memory can be any suitable memory such as for example a memory register or RAM (random access memory). It will be understood that the memory 13 and the processing unit 12 can be part of the same device (e.g. wearable device or wristband) or can be implemented as or in separate devices.

Fig. 3 shows an embodiment of a wearable device 50 wearable by user. In this embodiment, the wearable device 50 is implemented in the form of a smart watch. The smart watch comprises a wristband 53 and a casing 54. The wristband 53 can loop around the wrist of the user. It will be understood that a wearable device could also be worn around another suitable part of the body such as the ankle foot or hand or may be adapted for attachment to other parts of the body, e.g. in the form of a patch.

The wearable device 50 can comprise the proposed system 100 for bio-feedback to a user. In this way a corresponding system 100 can be provided in an unobtrusive and wearable format. Alternatively, the wearable device 50 may only comprise the sensing unit 20, in this embodiment a skin conductance sensor 20. The device 10 of the system 100 may be located at the remote location or implemented in a remote device (e.g. a remote computer, smartphone or patient monitor).

The sensor 20 in this embodiment comprises a first and a second skin conductance electrode 51, 52 in combination with a skin conductance measuring unit (not shown). In the embodiment of Fig. 2, two skin conductance electrodes 51, 52 are integrated into the casing 54 of the wearable device, however is also possible to integrate them for example into the wristband 53 so that they contact the underside of the wrist. The skin conductance electrodes 51, 52 can be arranged so as to contact the upper side of the wrist when the user wears the wearable device 50. An exemplary implementation of a wearable device comprising a skin conductance sensor is the Philips discreet tension indicator DTI-4 or DTI-5.

The skin conductance sensor 20 is adapted to measure the skin conductance of the user 2 between the skin conductance electrodes 51, 52. For this purpose, the skin conductance measuring sensor may comprise a voltage generator for applying a voltage between the at least two skin conductance electrodes, a sensing unit for sensing a current between the at least two electrodes, and/or a calculating unit for calculating the skin conductance based on the sensed current. The measured skin conductance over time forms, in this embodiment, the skin conductance trace (or data). The skin conductance trace (or data) may for example be stored in a memory of the wearable device 50, or may be transmitted (wirelessly or through a wire or signal line) to an external unit.

The skin conductance measuring sensor 20 and/or the device 10 (as shown in Fig. 2) may be integrated into the casing 54 of the wearable device 50. The wearable device 50 can further comprise a transmitter for transmitting data (such as the skin conductance measurements, the user instructions and/or the bio-feedback) over a wireless or wired communication link, e.g. using Low Energy Bluetooth or a similar method. However, it will be understood that the device 10 or processing unit 12 can also be implemented as or in separate parts or devices and that the wearable device 50 then transmits the skin conductance measurements to the separate part or device via the transmitter. Further, the user interface 30 may be integrated into the wearable device 50 as well.

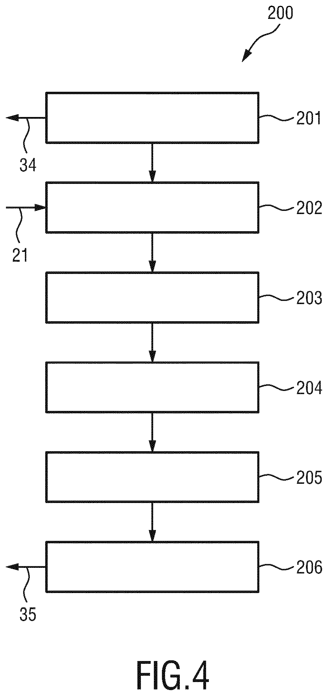

Fig. 4 shows a flow chart of an embodiment of a method 200 for providing bio-feedback to a user according to the present invention. The processing unit 12 of the device 10 shown in Fig. 1 may carry out this method 200.

In a first step 201 user instructions 34 indicating one or more actions to follow by the user are generated. For instance, the user may be instructed by displaying a certain text and/or graphic on the display screen 31 or by issuing corresponding audible instructions via a loudspeaker of the user interface 30 to take a certain position, hold the hands in a certain position and to breath in a certain rhythm and/or at a certain type of breathing (in which the belly is expanded when inhaling air) for a certain period. Subsequently, one or more further instructions may be provided, as e.g. done in a conventional Zen meditation session.

In a second step 202 skin conductance measurements 21 of a user are obtained, e.g. from the sensing unit 20, which acquired these measurements while the user followed the instructions given in the first step 201.

In a third step 203 one or more GSR storms are identified in the skin conductance trace of the obtained skin conductance measurements. For identifying a GSR storm various options exist, as will be explained below in more detail.

In a fourth step 204 a latency of a predetermined duration (in which the mind disturbances settle down and no further GSR storm shows) is identified after the end of an identified GSR storm, as will be explained below as well.

In a fifth step 205, if at least one GSR storm followed by a latency has been identified, bio-feedback information is generated providing bio-feedback related to the one or more actions followed by the user. Said bio-feedback may e.g. be meditation depth information and/or a meditation depth score.

In a sixth step 206 the generated bio-feedback information 35 is output to the user, e.g. in text form or in any other subtle form that can be percepted by the user and that does not disturb the state.

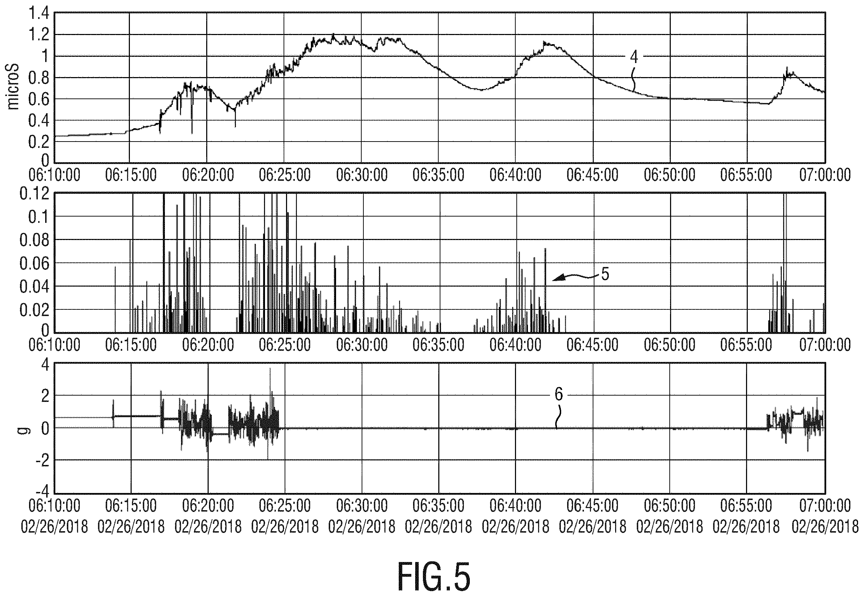

The GSR storm patterns in skin conductance traces of sleep can also be found in skin conductance traces measured during a Zen meditation. In Fig. 5 an example skin conductance trace 4 of a Zen meditation from 6:24 hrs until 6:57 hrs, the relative peak heights 5 of peaks in the skin conductance trace 4 and an accelerometer output signal 6 are shown. The skin conductance trace shows two GSR storms from 6:24 hrs until 6:35 hrs and from 6:37 hrs until 6:43 hrs. Analogous to slow wave sleep periods during a sleep period GSR storms occur at the beginning of the meditation period and settle down. In meditation terms (e.g. in line with the Aphorisms of Patanjali) a clear mind is obtained as soon as the mind disturbances settle down.

In the example of Fig. 5 a clear mind is obtained when the second GSR storm ends after about 18 minutes of meditation. The data processing unit 12 can detect and quantify the skin conductance peaks and signal the occurrence of slow (delta) brain frequencies to the meditator, or more advantageously the end of the GSR storms, indicating a clear mind state. A three to five-minute latency after the end of a GSR storm is preferably used to enable the detection of a new GSR storm.

The time and relative height or steepness of the individual GSR storm peaks can e.g. be calculated by taking the difference of logarithmic values of skin conductance measurement i and measurement i+1. Optionally, the value can be multiplied with the sampling:

$$steepness_{normalized, \log} = \left(10 \log\left(SC_i\right) - 10 \log\left(SC_{i+1}\right)\right) \cdot f$$

wherein $SC_i$ denotes a sample value of the skin conductance signal trace at sample i; $SC_{i+1}$ denotes a sample value of the skin conductance signal trace at a subsequent sample i+1; and f denotes the sampling frequency. The sampling frequency is optional in the aforementioned formulae. For such a log-calculation, all negative values can be set to zero, leaving only the rising edges of the skin conductance trace. Instead of steepness, it can also be called the first time derivative. Optionally, a normalized maximum rising edge slope of each of a plurality of respective skin conductance peaks can be identified and used for further processing.

In another embodiment, aside from a minimum rising edge duration criterion required for identifying a rising edge (i.e. the rising edge must be given for a minimum duration), zero crossings of the first derivative of the skin conductance signal may be used to detect a peak.

From the peak times the peak occurrence frequency can be obtained. Various criteria for identifying a GSR storm may be used. One criterion is that the peak frequency exceeds 0.2Hz for the entire duration of the GSR storm, more generally if the peak frequency of the peaks in the skin conductance trace exceeds a predetermined frequency threshold in the time period. A frequency in the range of 0.05 Hz to 1 Hz, in particular in the range of 0.1 Hz to 0.3 Hz, may hereby be used as predetermined frequency threshold.

Another criterion for detecting a GSR storm may be the if number of peaks in the skin conductance trace exceeds a predetermined threshold number in the time period. A number in the range of 3 to 60, in particular in the range of 6 to 18, may hereby be used as predetermined threshold number for a time period of one minute.

The duration of the GSR storm shall be in the range of 2 to 6 minutes, in particular in the range of 3 to 6 minutes. Further, a minimum number of subsequent peaks may be provided in the skin conductance trace to qualify as a GSR storm.

In a practical implementation the criterion may be used that there shall be at least 3 peaks/minute and a minimum number of 45 subsequent peaks to qualify as a GSR storm.

In a practical embodiment the disclosed method may be implemented on a smartphone which is usually the device that conveys information and communication to the user. A dedicated application ("app") can monitor the amount of information and communication to which the user has been exposed. The wearable skin conductance sensor is capable to calculate a stress level that can be linked to the contextual information from the smartphone. When a preset limit of for example 3 hours of exposure or a cumulative stress exposure of for instance 1 hour of level 3 or higher is exceeded the slow brain wave induction app can be offered to restore attentional capacity (recovery of action potential of synapses). The app may use known Zen meditation techniques. The user can wear the Philips DTI-5 skin conductance sensor, follow the instructions from the smartphone, conveyed via earphones, and obtain feedback of failure or success by means of a suitable discreet audio signal generated by the GSR storm detection software. At least one GSR storm is needed to achieve the clear mind status. The end of the slow brain wave induction app may signaled when the preset latency of 3 to 5 minutes after the last GSR storm is reached. This end signal can again be conveyed as an audio signal via earphones connected to the smartphone.

The present invention may be used in the context of lifestyle coaching, smartwatches, attentional capacity recovery coaching, and/or meditation coaching.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope. number in the range of 3 to 60, in particular in the range of 6 to 18, may hereby be used as predetermined threshold number for a time period of one minute.

The duration of the GSR storm shall be in the range of 2 to 10 minutes, in particular in the range of 3 to 6 minutes. Further, a minimum number of subsequent peaks may be provided in the skin conductance trace to qualify as a GSR storm.

In a practical implementation the criterion may be used that there shall be at least 3 peaks/minute and a minimum number of 45 subsequent peaks to qualify as a GSR storm.

In a practical embodiment the disclosed method may be implemented on a smartphone which is usually the device that conveys information and communication to the user. A dedicated application ("app") can monitor the amount of information and communication to which the user has been exposed. The wearable skin conductance sensor is capable to calculate a stress level that can be linked to the contextual information from the smartphone. When a preset limit of for example 3 hours of exposure or a cumulative stress exposure of for instance 1 hour of level 3 or higher is exceeded the slow brain wave induction app can be offered to restore attentional capacity (recovery of action potential of synapses). The app may use known Zen meditation techniques. The user can wear the Philips DTI-5 skin conductance sensor, follow the instructions from the smartphone, conveyed via earphones, and obtain feedback of failure or success by means of a suitable discreet audio signal generated by the GSR storm detection software. At least one GSR storm is needed to achieve the clear mind status. The end of the slow brain wave induction app may signaled when the preset latency of 3 to 5 minutes after the last GSR storm is reached. This end signal can again be conveyed as an audio signal via earphones connected to the smartphone.

The present invention may be used in the context of lifestyle coaching, smartwatches, attentional capacity recovery coaching, and/or meditation coaching.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

## Claims

1. Device for providing bio-feedback to a user, said device comprising:

   an interface (11) configured to obtain skin conductance measurements of a user and to output user instructions and bio-feedback information; and
   a processing unit (12) configured to

- generate user instructions indicating one or more actions to follow by the user,
- identify one or more galvanic skin response, GSR, storms in a skin conductance trace of the skin conductance measurements obtained during the execution by the user of the user instructions, wherein a GSR storm is identified based on the number of peaks and/or the peak frequency in a time period having a duration in the range of 2 to 6 minutes,
- identify a latency of a predetermined duration without a further GSR storm after the end of an identified GSR storm, and
- generate, if at least one GSR storm followed by a latency has been identified, bio-feedback information providing bio-feedback related to the one or more actions followed by the user.

2. Device as claimed claim 1,
wherein the processing unit (12) is configured to generate a meditation depth information indicating if at least one GSR storm followed by a latency has been identified as bio-feedback information.

3. Device as claimed in any one of the preceding claims,
wherein the processing unit (12) is configured to identify a GSR storm in the skin conductance trace by detecting if the peak frequency of the peaks in the skin conductance trace exceeds a predetermined frequency threshold in a time period.

4. Device as claimed in claim 3,
wherein the processing unit (12) is configured to use a frequency in the range of 0.05 Hz to 1 Hz, in particular in the range of 0.1 Hz to 0.3 Hz, as predetermined frequency threshold.

5. Device as claimed in any one of the preceding claims,
wherein the processing unit (12) is configured to identify a GSR storm in the skin conductance trace by detecting if the number of peaks in the skin conductance trace exceeds a predetermined threshold number in a time period.

6. Device as claimed in claim 5,
wherein the processing unit (12) is configured to use a number in the range of 3 to 60, in particular in the range of 6 to 18, as predetermined threshold number for a time period of one minute.

7. Device as claimed in claim 2, 3, 5 or 6,
wherein the processing unit (12) is configured to use a duration in the range of 3 to 6 minutes as time period.

8. Device as claimed in any one of the preceding claims,
wherein the processing unit (12) is configured to use a duration in the range of 2 to 10 minutes, in particular in the range of 3 to 6 minutes, as latency.

9. Device as claimed in any one of the preceding claims,
wherein the processing unit (12) is configured to generate user instructions indicating one or more meditation actions to follow by the user and/or a meditation depth score based on the detection of peaks in the skin conductance trace, the meditation depth score indicating a meditation state of the user.

10. System for providing bio-feedback to a user, said system comprising:

- a sensing unit (20) configured to acquire skin conductance measurements of the user,
- a device (10) as claimed in any one of the preceding claims configured to generate user instructions and to generate bio-feedback information based on the skin conductance measurements, and
- a user interface (30) configured to output the user instructions and the bio-feedback information.

11. System as claimed in claim 10,

wherein the sensing unit (20) is a wearable skin conductance sensor and/or
wherein the user interface (30) comprises one or more of a display screen (31) and/or a loudspeaker (32) and/or a communication unit (33) configured to communicate the user instructions and/or the bio-feedback information to another device (40).

12. Computer-implemented method for providing bio-feedback to a user, said method comprising:

- obtaining skin conductance measurements of a user and to output user instructions and bio-feedback information;
- generating user instructions indicating one or more actions to follow by the user,
- identifying one or more galvanic skin response, GSR, storms in a skin conductance trace (4) of the skin conductance measurements (21) obtained during the execution by the user of the user instructions, wherein a GSR storm is identified based on the number of peaks and/or the peak frequency in a time period having a duration in the range of 2 to 6 minutes,
- identifying a latency of a predetermined duration without a further GSR storm after the end of an identified GSR storm, and
- generating, if at least one GSR storm followed by a latency have been identified, bio-feedback information providing bio-feedback related to the one or more actions followed by the user.

13. Computer program comprising program code means for causing a computer to carry out the steps of the method as claimed in claim 12 when said computer program is carried out on the computer.

**Patentansprüche**

1. Vorrichtung zum Bereitstellen von Bio-Feedback für einen Benutzer, wobei die Vorrichtung Folgendes umfasst: Eine Schnittstelle (11), die dazu konfiguriert ist, Messungen der Leitfähigkeit der Haut eines Benutzers zu erhalten und Benutzeranweisungen und Bio-Feedback-Informationen auszugeben; und eine Verarbeitungseinheit (12), die dafür konfiguriert ist:

   - Benutzeranweisungen zu erstellen, die eine oder mehrere vom Benutzer zu befolgende Aktionen angeben,
   - Identifizieren einer oder mehrerer galvanischer Hautreaktionen, GSR, Stürme in einer Hautleitfähigkeitsspur der Messungen zur Hautleitfähigkeit, die der Nutzer während der Ausführung der Anweisungen für den Nutzer erhalten hat, und in denen ein GSR-Sturm anhand der Anzahl der Spitzen und/oder der Spitzenhäufigkeit in einem Zeitraum mit einer Dauer im Bereich von 2 bis 6 Minuten identifiziert wird,
   - Identifizieren einer Latenz einer vorbestimmten Dauer ohne einen weiteren GSR-Sturm nach dem Ende eines identifizierten GSR-Sturms und
   - Erzeugen, wenn mindestens ein GSR-Sturm gefolgt von einer Latenz identifiziert wurde, von Biofeedback-Informationen, die Bio-Feedback in Bezug auf eine oder mehrere vom Benutzer ausgeführte Aktionen darbieten.

2. Vorrichtung wie in Anspruch 1, wobei die Verarbeitungseinheit (12) dazu konfiguriert ist, eine Meditationstiefen-Information zu erzeugen, die angibt, ob mindestens ein GSR-Sturm gefolgt von einer Latenzzeit als Bio-Feedback-Informationen identifiziert wurde.

3. Vorrichtung wie in einem der vorangegangenen Ansprüche, wobei die Verarbeitungseinheit (12) dazu konfiguriert ist, einen GSR-Sturm in der Haut-Leitfähigkeitsspur zu identifizieren, indem sie erkennt, wenn die Spitzenfrequenz der Spitzen in der Hautleitfähigkeitsspur einen vorbestimmten Häufigkeitsschwellenwert in einem Zeitraum überschreitet.

4. Vorrichtung nach Anspruch 3, wobei die Verarbeitungseinheit (12) so konfiguriert ist, dass sie eine Frequenz im Bereich von 0,05 Hz bis 1 Hz und insbesondere im Bereich von 0,1 Hz bis 0,3 Hz als vorgegebenen Frequenzschwellenwert nutzt.

5. Vorrichtung wie in einem der vorangegangenen Ansprüche, wobei die Verarbeitungseinheit (12) dazu konfiguriert ist, einen GSR-Sturm in der Haut-Leitfähigkeitsspur zu identifizieren, indem sie erkennt, wenn die Anzahl der Spitzen in der Haut-Leitfähigkeitsspur eine vorbestimmte Schwellenzahl in einem Zeitraum überschreitet.

6. Vorrichtung nach Anspruch 5, wobei die Verarbeitungseinheit (12) so konfiguriert ist, dass sie eine Anzahl im Bereich von 3 bis 60, insbesondere im Bereich von 6 bis 18, als vorgegebene Schwellenzahl für einen Zeitraum einer Minute nutzt.

7. Vorrichtung wie in den Ansprüchen 2, 3, 5 oder 6, wobei die Verarbeitungseinheit (12) so konfiguriert ist, dass sie eine Dauer im Bereich von 3 bis 6 Minuten als Zeitraum nutzt.

8. Vorrichtung wie beansprucht in einem der vorangegangenen Ansprüche, wobei die Verarbeitungseinheit (12) so konfiguriert ist, dass sie eine Dauer im Bereich von 2 bis 10 Minuten und insbesondere im Bereich von 3 bis 6 Minuten als Latenz nutzt.

9. Vorrichtung wie beansprucht in einem der vorangegangenen Ansprüche, wobei die Verarbeitungseinheit (12) dazu konfiguriert ist, Anweisungen für den Nutzer zu erzeugen, welche eine oder mehrere Meditationshandlungen anzeigen, denen der Benutzer folgen soll, und/oder eine darauf basierende Meditationstiefenbewertung auf Grundlage der Detektion von Spitzen in der Hautleitfähigkeitsspur, der Meditationstiefenwert, der einen Meditations-Zustand des Benutzers anzeigt.

10. Vorrichtung zum Bereitstellen von Bio-Feedback für einen Benutzer, wobei das besagte System umfasst:

- eine Sensoreinheit (20), die dazu konfiguriert ist, Messungen der Leitfähigkeit der Haut des Benutzers zu erfassen,
- eine Vorrichtung (10) wie beansprucht in einem der vorangegangenen Ansprüche, die dafür konfiguriert ist, eine Gebrauchsanweisung und Bio-Feedback-Informationen basierend auf Messungen des Hautleitwerts zu erzeugen, und
- eine Benutzerschnittstelle (30), die dazu konfiguriert ist, die Benutzeranweisungen und die Bio-Feedback-Informationen auszugeben.

11. System nach Anspruch 10, wobei die Sensoreinheit (20) ein tragbarer Hautleitfähigkeitssensor ist und/oder wobei die Benutzerschnittstelle (30) einen oder mehrere Anzeigebildschirme (31) und/oder einen Lautsprecher (32) und/oder eine Kommunikationseinheit (33) umfasst, die dazu konfiguriert sind, die Benutzeranweisungen und/oder die Bio-Feedback-Informationen an ein anderes Gerät (40) zu übermitteln.

12. Durch Computer verwirklichtes Verfahren zum Bereitstellen von Bio-Feedback für einen Benutzer, wobei das besagte Verfahren umfasst:

- Erhalten von Hautleitwertmessungen eines Benutzers und Ausgeben von Benutzeranweisungen und Bio-Feedback-Informationen;
- Erzeugen von Benutzeranweisungen, die eine oder mehrere vom Benutzer zu befolgende Aktionen angeben,
- Identifizieren einer oder mehrerer galvanischer Hautreaktionen, GSR, Stürme in einer Haut-Leitfähigkeitsspur (4) der Messungen zur Hautleitfähigkeit (21) die während der Ausführung der Anweisungen für den Nutzer durch diesen erhalten wurden, und in denen ein GSR-Sturm anhand der Anzahl der Spitzen und/oder der Spitzenhäufigkeit in einem Zeitraum mit einer Dauer im Bereich von 2 bis 6 Minuten identifiziert wird,
- Identifizieren einer Latenz einer vorbestimmten Dauer ohne einen weiteren GSR-Sturm nach dem Ende eines identifizierten GSR-Sturms und
- Erzeugen, wenn mindestens ein GSR-Sturm gefolgt von einer Latenz identifiziert wurde, von Bio-feedback-Informationen, die Bio-Feedback in Bezug auf eine oder mehrere vom Benutzer ausgeführte Aktionen darbieten.

13. Computerprogramm, das Programmcodemittel umfasst, um einen Computer dazu zu veranlassen, die Schritte des Verfahrens gemäß Anspruch 12 auszuführen, wenn das besagte Computerprogramm auf dem Computer ausgeführt wird.

**Revendications**

1. Un dispositif pour fournir une rétroaction biologique à un utilisateur, ledit dispositif comprend:

une interface (11) configurée pour obtenir des mesures de conductance cutanée d'un utilisateur et pour fournir des instructions à l'utilisateur et des informations sur la rétroaction biologique; et
une unité de traitement (12), configurée pour

- générer des instructions de l'utilisateur indiquant une ou plusieurs actions à suivre par l'utilisateur,
- identifier une ou plusieurs réponses galvaniques de la peau, des GSR, des variations dans une trace de conductance de la peau des mesures de conductance de la peau obtenues lors de l'exécution par l'utilisateur des instructions utilisateur, où une variation GSR est identifiée à partir du nombre de pics et/ou la fréquence de pics dans un temps donné ayant une durée allant de 2 à 6 minutes,
- identifier une latence d'une durée prédéterminée sans une variation GSR après la fin d'une variation GSR identifiée, et

- la production, d'au moins une variation GSR suivie par une latence qui a été identifiée, une information de rétroaction biologique fournissant une rétroaction biologique liée à une ou plusieurs actions suivies par l'utilisateur.

2.  Un dispositif comme revendiqué selon la revendication 1, où l'unité de traitement (12) est configurée pour générer une information de profondeur de méditation indiquant si au moins une variation GSR suivie par une latence a été identifiée en tant qu'information de rétroaction biologique.

3.  Un dispositif comme revendiqué selon l'une quelconque des revendications précédentes, où l'unité de traitement (12) est configurée pour identifier une variation GSR dans la trace de conductance cutanée en détectant si la fréquence de pics dans la trace de conductance cutanée dépasse un seuil de fréquence prédéterminée sur une période.

4.  Un dispositif comme revendiqué selon la revendication 3, où l'unité de traitement (12) est configurée pour utiliser une fréquence dans une plage allant de 0,05 Hz à 1 Hz, en particulier dans une plage allant de 0,1 Hz à 0,3 Hz, comme seuil de fréquence prédéterminée.

5.  Un dispositif comme revendiqué selon l'une quelconque des revendications précédentes, où l'unité de traitement (12) est configurée pour identifier une variation GSR dans la trace de conductivité cutanée en détectant si le nombre de pics dans la trace de conductivité cutanée dépasse un nombre de seuils prédéterminés sur une période.

6.  Un dispositif comme revendiqué selon la revendication 5, où l'unité de traitement (12) est configurée pour utiliser un nombre dans la plage allant de 3 à 60, en particulier dans une plage allant de 6 à 18, comme nombre seuil prédéterminé sur une période d'une minute.

7.  Un dispositif comme revendiqué dans les revendications 2,3, 5 ou 6, où l'unité de traitement (12) est configurée pour utiliser une durée dans une plage allant de 3 à 6 minutes comme période.

8.  Un dispositif comme revendiqué selon l'une quelconque des revendications précédentes, où l'unité de traitement (12) est configurée pour utiliser une durée allant de 2 à 10 minutes, en particulier dans une plage allant de 3 à 6 minutes, comme latence.

9.  Un dispositif comme revendiqué selon l'une quelconque des revendications précédentes, où l'unité de traitement (12) est configurée pour générer des instructions de l'utilisateur indiquant une ou plusieurs actions de méditations à suivre par l'utilisateur et/ou un score de profondeur de méditation fondée sur la détection des pics dans la trace de conductivité cutanée, le score de profondeur de méditation indiquant un état de méditation chez l'utilisateur.

10. Un système pour fournir un biofeedback à un utilisateur, ledit système comprend:

    - une unité de détection (20) configurée pour obtenir des mesures de conductance de la peau de l'utilisateur,
    - un dispositif (10) comme revendiqué selon l'une quelconque des revendications précédentes configurées pour générer des instructions aux utilisateurs et pour générer des informations de rétroaction biologique fondées sur les mesures de conductance de la peau, et
    - une interface utilisateur (30) configurée pour afficher les instructions utilisateurs et les informations de rétroaction biologique.

11. Un système comme revendiqué dans la revendication 10, où l'unité de détection (20) est un capteur de conductance cutanée portable et/ou où l'interface utilisateur (30) comprend un ou plusieurs écrans d'affichage (31) et/ou un haut-parleur (32) et/ou une unité de communication (33) configurée pour communiquer des instructions utilisateurs et/ou des informations de rétroaction biologique à un autre dispositif (40).

12. Une méthode exécutée par un ordinateur pour fournir une rétroaction biologique à l'utilisateur, ladite méthode comprend:

    - l'obtention des mesures de conductance cutanée d'un utilisateur et pour les instructions de sortie de l'utilisateur et les informations de rétroaction biologique;
    - la production d'instructions utilisateur indiquant une ou plusieurs actions à suivre par un utilisateur,

- l'identification d'une ou plusieurs réponses galvaniques de la peau, GSR, des variations dans la trace de conductance de la peau (4) des mesures de la conductance de la peau (21) obtenues lors de l'exécution par l'utilisateur des instructions aux l'utilisateur, où une variation GSR est identifiée à partir du nombre de pics et/ou de pics de fréquence sur une période possédant une durée allant de 2 à 6 minutes,

- l'identification d'une latence d'une durée prédéterminée sans une variation GSR après la fin d'une variation GSR identifiée, et

- la production, d'au moins une variation GSR suivie par une latence ayant été identifié, les informations de rétroaction biologique fournissent une rétroaction biologique liée à une ou plusieurs actions suivies par l'utilisateur.

13. Le programme informatique comprend des moyens de codage informatique pour faire en sorte que le système exécute les étapes suivantes de la méthode comme revendiquée dans la revendication 12 lorsque ledit programme informatique est exécuté par l'ordinateur.

FIG.1

FIG.2

FIG.3

FIG.4

FIG.5

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• US 20140288401 A1